(19) Europäisches Patentamt
European Patent Office
Office européen des brevets

(11) Publication number: **0 247 900 B1**

# (12) EUROPEAN PATENT SPECIFICATION

(45) Date of publication of patent specification: **18.12.91 Bulletin 91/51**

(51) Int. Cl.$^5$: **A61K 31/505**, A61K 47/00

(21) Application number: **87304797.1**

(22) Date of filing: **29.05.87**

## (54) Topical methotrexate preparation for the treatment of hyperproliferative epithelial disease.

(30) Priority: **30.05.86 US 868936**

(43) Date of publication of application: **02.12.87 Bulletin 87/49**

(45) Publication of the grant of the patent: **18.12.91 Bulletin 91/51**

(84) Designated Contracting States: **AT BE CH DE ES FR GB IT LI NL SE**

(56) References cited:
**EP-A- 0 129 284**
**EP-A- 0 164 998**
**GB-A- 1 553 310**
**GB-A- 2 143 433**

(56) References cited:
**CHEMICAL ABSTRACTS, vol. 84, no. 19, May 10, 1976, pages 11-12, ref.no. 130110f; Columbus; Ohio, US J.L. McCULOUGH ET AL::"Factors affecting human percutaneous penetration of methotrexate and its analogs in vitro." & J.INVEST. DERMATOL.1976, 66 (2), 103-7 (cat. D)**
**ACTA DERM. VENEROL (Stock) 66: 515-519 (1986)**
**ARCH. DERM. 87: 258-265 (1963)**
**ARCH. DERM. 100: 99-105 (1969)**
**ARCH. DERM. RES.253 : 77-84 (1975)**
**J. INVEST. DERMATOL. 66: 103-107 (1976)**
**DT-05 2304981**

(73) Proprietor: **THE REGENTS OF THE UNIVERSITY OF CALIFORNIA**
**2199 Addison Street**
**Berkeley, California 94720 (US)**

(72) Inventor: **Weinstein, Gerald D.**
**19101 Croydon Terrace**
**Irvine California 92715 (US)**
Inventor: **McCullough, Jerry L.**
**30 Birdsong**
**Irvine California 92713 (US)**

(74) Representative: **Goldin, Douglas Michael et al**
**J.A. KEMP & CO. 14, South Square Gray's Inn**
**London WC1R 5EU (GB)**

**EP 0 247 900 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical Field

The present invention relates to topical preparations containing methotrexate or derivatives thereof in the treatment of hyperproliferative epithelial diseases, such as psoriasis.

### Background of the Invention

The invention described herein was made in the course of work under a grant or award from the National Institutes of Health, U.S. Department of Health and Human Services.

Epithelial diseases (epidermal and mucosal) are a major health problem. Of particular concern are those characterized by abnormal (increased) rates of cell turnover, referred to herein as hyperproliferative epithelial diseases. Examples of hyperproliferative epithelial diseases include psoriasis, cutaneous tumors primary to the skin (basal cell carcinoma, squamous cell carcinoma, melanoma, mycosis fungoides, Bowen's disease), viruses (warts, herpes simplex, condyloma acuminata), premalignant and malignant diseases of the female genital tract (cervix, vagina, vulva) and premalignant and malignant diseases of mucosal tissues (oral, bladder, rectal). The most common such disease is psoriasis. Recent estimates have indicated that there are 1 to 3 million persons in the United States with psoriasis, and approximately 150,000 to 250,000 new cases reported annually. The prevalence rate of psoriasis in the United States is between 3 and 4% of the population, with similar prevalence rates in other countries. There is a great need, therefore, for an effective therapeutic regimen.

A variety of therapies are currently used to treat psoriasis, including dialysis, chemotherapy (topical and systemic) and photochemotherapy (topical and systemic). Topical chemotherapy is probably the most widely used, employing agents such as tar, retinoids, anthralin, corticosteroids, and antimetabolites.

At present, the most severe cases of psoriasis are being treated with systemic photochemotherapy or PUVA (psoralen + UVA). There is a risk, however, that such therapy causes direct changes in the DNA structure of the patient's cells. Thus, such therapeutic approaches are potentially mutagenic and carcinogenic. R.S. Stern et al, (1979) New England Journal of Medicine. 300:809-813. Systemic chemotherapy employing methotextrate (MTX) has also been used effectively in the treatment of severe or extensive psoriasis. MTX is administered orally or by injection, but its chronic use is associated with significant side-effects, particularly liver damage. Weinstein, (1977) Ann.Int. Med. 86:199.

The majority of patients with psoriasis have minimal or moderate involvement. Thus, the risks associated with PUVA or systemic chemotherapy with MTX are not warranted. These patients are generally treated with topical steroids, a form of therapy which is either initially or eventually ineffective. There is a need, therefore, for an effective and safe form of local therapy for the majority of patients afflicted with hyperproliferative skin disease.

Since MTX has been demonstrated to be an effective systemic chemotherapeutic drug in the treatment of psoriasis, van Scott et al, (1964) Arch. Derm. 89:550; Rees et al, (1967) Arch. Derm. 95:2, it has been suggested that MTX might be developed into an effective chemotherapeutic drug for topical delivery. Topical application of MTX would be highly desirable because the harmful side effects of systemic administration would be minimized since the drug would be applied only to afflicted tissue; unafflicted tissue, such as the liver, would be exposed to little or no drug. Several prior art studies applying MTX topically, however, reported that the drug was ineffective. See Weinstein et al, (1981) Arch. Derm. 117:388; Weinstein in Pharmacology and the Skin; Advances in Biology of the Skin, Vol. XII pp. 287-202 (W. Montagna, RB Stoughton & EJ van Scott eds. 1969); van Scott et al, (1959) J. Invest. Derm. 33:357; Nurse, (1963) Arch. Derm. 87:168; Comaish et al, (1969) Arch. Derm. 100:99; Stewart et al, (1972) Arch. Derm. 106:357. For example, in Weinstein et al. (1981), methotrexate in a vehicle containing 2.5% of the penetration-enhancing agent C-10-methylsulfoxide was clinically evaluated. Six patients were treated daily for nine days with vehicle formulations containing 0.1% and 0.5% MTX. There was no significant improvement in any of the patients.

Other studies have shown that topical MTX preparations produced negligible percutaneous penetration of human skin in vitro and in vivo. McCullough et al, (1976) J. Invest. Derm. 66:103; Stewart et al, (1972) supra; Comaish et al, (1969) supra. Thus, it has been hypothesized that MTX either (a) does not sufficiently penetrate the skin, (b) acts systemically at a site distant from the afflicted tissue, or (c) is converted to an active form at a site distant from the afflicted tissue. See Weinstein et al. (1976) J.Invest. Derm. 67:26.

Additional studies of interest in the mechanism of MTX action are Weinstein et al, (1971) Arch Derm 104:236 and Newberger et al, (1978) J Invest Derm 70;183.

invention may also be used to treat other hyperproliferative epithelial diseases, including viral diseases such as herpes simplex and warts, and cutaneous malignancies primary to the skin (e.g., squamous cell carcinoma, basal cell carcinoma, melanoma) or metastatic lesions of internal malignancies present on the skin.

A major component of compositions for topical application will be a topical carrier. The term "topical carrier" as used herein refers to carrier materials suitable for topical applications of drugs, such as MTX, and include any such materials known in the cosmetic and medical arts. Suitable carriers include, for example, water, liquid alcohols, liquid glycols, liquid polyalkylene glycols, liquid esters, liquid amides, liquid protein hydrolysates, liquid alkylated protein hydrolysates, liquid lanolin and lanolin derivatives, and like materials commonly employed in cosmetic and medicinal compositions. Exemplary carriers herein include: alcohols, including both monohydric and polyhydric alcohols, e.g., ethanol, isopropanol, glycerol, sorbitol, 2-methoxyethanol, diethylene glycol, ethylene glycol, hexylene glycol, mannitol, and propylene glycol; ethers such as diethyl or dipropyl ether; polyethylene glycols and methoxypolyoxyethylenes; carbowaxes having molecular weights ranging from 200 to 20,000; polyoxyethylene glycerols; polyoxyethylene; sorbitols; and stearoyl diacetin. Oil-in-water emulsions, such as cold cream bases, can also be used. The topical carriers described herein also include various agents and ingredients commonly employed in dermatological and cosmetic ointments and lotions. For example, perfumes, thickening agents such as carboxymethylcellulose, stabilizers, surfactants, emolients, coloring agents, and the like can be present in the carrier.

Topical carriers, such as ointments, creams, salves, jellies, pastes and lotions, are collectively referred to herein as "viscous topical medicament carriers." In general, viscous topical medicament carriers are a lubricating or moisturizing type which do not dry the patient's skin, as do carriers comprised primarily of water and alcohol. An example of a viscous topical medicament carrier is Eucerin™ cream, available from Beiersdorf Inc., S. Norwalk, CN, which is an unscented moisturizing formula for dry skin containing water, petroleum, mineral oil, mineral wax, wool wax alcohol, and 2-bromo-2-nitropropane-1,3-diol. Other viscous topical medicament carriers are "Vehicle N" (Neutrogena Corp., Los Angeles, CA), Aquaphor ointment base (Dule Laboratories, South Norwalk, CN), Unibase ointment base (Parke-Davis, Detroit, MI), and petrolatum. Other types of topical carriers are known, such as mixtures of one or more of ethanol, isopropanol, N-methylpyrrolidone (NMP), and water.

Another necessary element in topical therapeutic compositions of the present invention is a skin penetration agent. The preferred penetration agents are the 1-substituted-azacycloheptan-2-ones disclosed in U.S. Patent Nos. 3,989,816, 4,405,616, and 4,316,893, the disclosures of which are incorporated herein by reference. These compounds have the general formula:

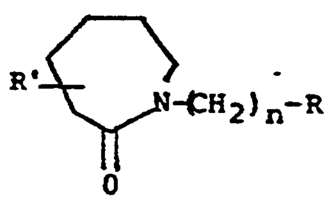

wherein R′ is selected from the group consisting of H and alkyl having 1-4 carbon atoms, R is selected from the group consisting of an alkyl of 1-18 carbon atoms and phenyl, and n is 0 or a positive integer from 1-10. Examples include 1-methyl-azacycloheptan-2-one, 1-octyl-azacycloheptan-2-one, and 1-nonyl-azacycloheptan-2-one. A particularly preferred member of this class is 1-dodecyl-azacycloheptan-2-one, which is available under the trademark Azone from Nelson Research & Development Co., Irvine, CA. The concentration of these penetration agents will preferably be between 0.1% and 10%, most preferably between 0.1% and 5%, by weight of the topical carrier.

The ultimate dosage delivered to the afflicted tissue by topical application will depend upon the concentration of MTX in the topical carrier, the amount of the topical composition which is applied to the afflicted tissue, the frequency of application, as well as other factors. In general, concentrations of MTX can range from 0.1% to 10% by weight of the composition. Concentrations outside of this range could be efficacious depending on other factors. Typical formulations, however, will have concentrations of MTX of 0.5%, 1%, 1.5%, or 2%, all by weight of the composition. A concentration is selected so that a particular topical formulation will deliver a therapeutically effective amount of MTX to the basal epidermal cells, as described above. The determination of appropriate concentrations for MTX, as well as the skin penetration agent, is within the skill of the ordinary artisan in light of the present disclosure. Various formulations can be screened easily in the in vitro and in vivo models described in the examples.

A particularly preferred composition for topical application is one comprised of methotrexate and Azone in a viscous topical medicament carrier. The superior skin penetration properties of Azone generally allows for

## Summary of the Invention

It is an object of the present invention to provide a composition for use in the treatment or therapy of hyperproliferative epithelial diseases, such as psoriasis.

It is also an object of the present invention to provide a topical preparation for the treatment of hyperproliferative epithelial diseases.

Another object of the present invention is to provide a composition for use in a therapeutic method for the treatment of hyperproliferative epithelial diseases which avoids the disadvantages associated with current PUVA therapy or systemic chemotherapy.

Yet another object of the present invention is to provide a composition for use in a topical therapeutic method for hyperproliferative epithelial diseases.

A further object of the present invention is to provide a topical formulation which allows for enhanced precutaneous penetration of methotrexate or derivatives thereof.

These and other objects and advantages of the present invention will be readily apparent to those skilled in the art from the embodiments set forth below.

In one embodiment, the present invention is directed to a composition for topical application that is therapeutic for a hyperproliferative epithelial disease comprising: (a) a topical carrier containing an effective amount of a skin penetration agent that allows methotrexate to penetrate to the basal epidermal cells of a mammal in sufficient quantity to inhibit DNA synthesis in said basal epidermal cells; and (b) a therapeutically effective amount of a drug selected from methotrexate; 3′,5′-dichloromethotrexate; a mono- or dialkyl ester of methotrexate; and a mono- or dialkyl ester of 3′-5′-dichloromethotrexate.

In another embodiment, the present invention is directed to a composition for use in the therapeutic treatment of a hyperproliferative epithelial disease. The treatment comprise topically administering to an area of mammalian skin afflicted by said hyperproliferative epithelial disease a therapeutically effective amount of methotrexate or a methotrexate derivative wherein said administration results in sufficient methotrexate or methotrexate derivative penetrating to the basal epidermal cells of said mammalian skin in sufficient quantity to inhibit DNA synthesis in said basal epidermal cells.

Other embodiments of the present invention will be readily apparent to those skilled in the art from the following disclosure.

## Detailed Description of the Invention

Methotrexate and its analogs have long been known to be therapeutically active against hyperproliferative epithelial diseases, such as psoriasis. While MTX is effective when administered systemically, the exposure of the entire body to the drug leads to serious side effects. Thus, the prior art tested methotrexate in topical formulations. These tests, however, failed to produce positive therapeutic results. This raised the possibility in the prior art that methotrexate's activity was tied to the mode of its administration; i.e., systemic.

Applicants, however, found that when administered intradermally, MTX produces the same biochemical effects (inhibition of psoriatic epidermal DNA synthesis) and biological actions (mitotic inhibition and damaged cell production) as systemic MTX. This led to the further finding that prior art topical formulations of MTX did not allow sufficient penetration of MTX into the skin, and that it was necessary to include a skin penetration agent which promotes the penetration of MTX to the basal epidermal cells. Thus, it has been found that MTX or its derivatives can be administered topically in an effective manner to skin afflicted with a hyperproliferative epithelial disease avoiding, therefore, the serious side effects associated with systemic MTX administration.

Methotrexate (MTX) and its derivatives effective against hyperproliferative epithelial diseases, such as psoriasis, shall be referred to collectively herein as methotrexate or MTX. Methotrexate and its analogs have long been recognized by the art as a group of drugs therapeutically active against hyperproliferative epithelial disease, such as psoriasis and malignant cells. (See McCullough et al. (1976) J. Invest. Derm. 66:103-107.) Derivatives of MTX include 3′,5′-dichloromethotrexate, monoalkyl esters of methotrexate, (including the dimethyl or diethyl esters of methotrexate), and monoalkyl and dialkyl esters of 3′5′-dichloromethotrexate (including the ethyl ester of 3′,5′-dichloromethotrexate and the diethyl ester of 3′,5′-dichloromethotrexate). The preferred monoalkyl and dialkyl esters preferably contain from 1 to 10 carbon atoms per alkyl group, and oftentimes from 1 to 5 carbon atoms per alkyl group.

The term "hyperproliferative epithelial disease" as used herein, means conditions of the skin that are characterized by epidermal cell proliferation or incomplete cell differentiation. These conditions may occur spontaneously or be induced by means external to the body, such as exposure to radiation or chemicals. Such diseases include psoriasis, cervical displasia and other pre-malignant lesions, and mycosis fungoides. Of particular concern to the present invention is the treatment of psoriasis. The topical compositions of the present

the use of lower concentrations of MTX in the formulations. A preferred formulation of the present invention is a topical composition comprising 0.1%, 0.5% or 1% MTX and 3% Azone in a viscous topical medicament carrier such as a gel. The gel carrier can contain polyethylene glycol (PEG 200) to solubilize MTX, isopropanol as a co-solvent for MTX, Carbopol 934P gelling agent, Tween-20 non-ionic emulsifier, triethanolamine to neutralize the gelling agent, and hydrochloric acid to present MTX as a combination of base and hydrochloride.

Topical administration of MTX may require repeated applications extending over several weeks or days. The exact number of applications and length of treatment will generally depend upon the concentration of MTX in the topical formulation, as well as the efficacy of the skin penetration agent. For example, in a topical formulation containing 1% methotrexate and 3% Azone, it is believed that a regimen of two applications per day to afflicted tissue for a period of 3-6 weeks should produce a therapeutic effect.

The following examples are provided for illustrative purposes only and are not intended to limit the scope of the invention in any way. Percentages are by weight unless otherwise noted.

Example I

In Vitro Percutaneous Penetration Assay with MTX and Vehicle N

Methotrexate was obtained from Lederle Laboratories, Pearl River, NY. Methotrexate (3',5',7-$^3$H)sodium (250 mCi/mmol) was obtained from Amersham and prior to use was purified by chromatography on thin-layer cellulose plates in 0.006 M potassium phosphate buffer, pH 6.0. Vehicle N was obtained from the Neutrogena Corporation, Los Angeles, CA.

The percutaneous penetration of MTX was measured in glass diffusion cells as previously described. McCullough et al. (1976) J. Invest. Derm. 66:103-107. Excised human skin obtained from abdominoplasty was used for these studies. First, 0.5 ml containing 10 µCi methotrexate-$^3$H and cold methotrexate to give a final concentration of 2% in various vehicles was applied to the epidermal surface (0.2 ml/cm$^2$). A total of 10 µg methotrexate was applied to each diffusion cell. The dermal reservoir contained phosphate buffered saline. Three diffusion cells were run for each vehicle. Diffusion cells were incubated with constant stirring at 28°C. Methotrexate penetration was followed at various time points up to 48 hr by liquid scintillation counting. The test solution was removed after the 48 hr incubation period, and the epidermal surface was rinsed three times with vehicle in which the drug being tested was dissolved. This process effectively removed all drug which had not penetrated into the stratum corneum. The epidermis was separated by heating the skin for 1 min on a hot plate at 60°C. MTX was extracted from the epidermis and dermis utilizing the techniques described by Werkheiser et al. (1962) J. Pharmacol. Exp. Ther. 137:162-166, which specifically disassociates MTX from cellular bound proteins. The stratum corneum was removed by repeated stripping with cellophane tape until the skin glistened.

The results are shown in Table 1. There was a significant increase in MTX penetration at both 24 and 48 hr in vehicle N as compared with water ($p < 0.01$) and n-decylmethylsulfoxide ($p < 0.01$). Vehicle N also produced a slight increase in epidermal MTX content compared to the other vehicles.

Table 1

| Vehicle | Penetration[a] 24 hr | | 48 hr | | Drug Content[a,b] Stratum Corneum | | Epidermis | |
|---|---|---|---|---|---|---|---|---|
| | µg | % | µg | % | µg | % | µg | % |
| Water | 6±2 | 0.06 | 15±5 | 0.15 | 9±5 | 0.09 | 0.1±0 | 0.001 |
| $C_{10}$MSO (2.5%) | 12±10 | 0.12 | 36±28 | 0.36 | 21±5 | 0.21 | 0.8±0.3 | 0.008 |
| Vehicle N | 50±11 | 0.50 | 124±10 | 1.24 | 16±8 | 0.16 | 1.5±0.5 | 0.01 |

[a]Mean values ± SD for 4 diffusion chambers.
[b]Samples taken after 48 hr incubations.
[c]Percent of applied dose.

Example II

In Vivo Study of MTX in Vehicle N

Deoxyuridine (6-$^3$H) (25 Ci/mmol) was obtained from New England Nuclear. Other materials were obtained

as described in Example I.

HRS/J albino hairless mice, age 2-3 months, were used for these experiments. For induction of the central fatty acid deficiency, 3-week-old animals were fed an essential fatty acid deficient diet (Teklad) for 60 days.

Topical MTX preparations at various concentrations or vehicle control in the amount of 0.05 ml were applied to the backskin area of 1.5 cm by 3 cm daily for 1 or 3 days. Six to ten mice were used for each treatment group. Tritiated deoxyuridine was utilized as a precursor for assaying the effects of methotrexate on the de novo pathway for epidermal DNA synthesis. The incorporation of tritiated deoxyuridine into DNA depends on the normal functioning of dihydrofolate reductase which is blocked by methotrexate. At selected time points after single or repeated topical MTX applications, mice were injected IP with tritiated deoxyuridine (50-100 μCi) for measurement of epidermal DNA synthesis in treated backskin to determine the local effect of MTX. DNA synthesis was simultaneously measured in untreated abdominal skin of Vehicle N and MTX-treated animals to determine systemic effects of MTX at a distant site. One hour after administration of isotope, animals were sacrificed by cervical dislocation, and treated backskin and untreated abdominal skin was obtained. Epidermis was removed after incubating the skin specimens for 1 hr at 37°C in 2 M sodium bromide. DNA was extracted and assayed using the technique described by Halprin et al. (1979) J. Invest. Derm. 73:359-363. Results of epidermal DNA synthesis were calculated as counts/minute/μg of DNA. The effects of MTX treatment on DNA synthesis of the back and abdomen were expressed as percentage of the DNA synthesis of Vehicle N treated back skin and untreated abdomin in control animals.

Three consecutive applications of Vechicle N in normal hairless mice control animals unexpectedly produced epidermal hyperplasia characterized by exfoliation, acanthosis, and hypergranulosis. Three daily applications of 2% MTX in Vehicle N to both normal and hyperproliferative essential fatty acid deficient hairless mouse skin produced marked epidermal atrophy in the areas where it was applied, without similarly effecting untreated abdominal skin.

Three daily applications of 2% MTX and Vehicle N suppressed epidermal DNA synthesis 50% and 75% in treated back skin compared to the Vehicle N treated control, at 54 and 72 hr, respectively. In this experiment, no significant ($p < 0.001$) systemic methotrexate effect was seen in the untreated abdominal skin at 54 hr (6 hr after the last treatment). By 72 hr, 24 hr after the last treatment, however, both local and systemic effects were obtained. Four daily applications of MTX resulted in total suppression of DNA synthesis in both the back skin and in the untreated abdominal skin.

Three daily applications of 1% MTX in Vehicle N also produced inhibition of DNA synthesis in treated skin 6 hr after the last treatment, while lower concentrations had no local or systemic effect. Topical MTX (0.5%) in N-decylmethylsulfoxide likewise had no effect on DNA synthesis in this model.

Epidermal DNA synthesis in the normal and essential fatty acid deficient hairless mouse has been a useful bioassay for topical chemotherapeutic drug activity against psoriasis. Lowe et al. (1977) Br. J. Dermatol. 96:155-162; Lowe et al. (1981) Arch. Dermatol. 117:394-398. Testing the topical MTX/DMSO preparation, previously shown to lack clinical activity in psoriasis (Weinstein et al. (1981) Arch. Dermatol. 117:388-393), showed no effect on epidermal DNA synthesis in the hairless mouse model. In this model, MTX in Vehicle N inhibited DNA synthesis and treated skin without a significant effect on distance skin 6 hr after the third application. A single drug application was ineffective. Twenty-four hr after the third application, DNA synthesis was inhibited at both the treated and distance skin sites presumably due to entry of MTX into the systemic circulation of the mouse.

Example III

In Vitro Study of MTX and Azone Formulations

In vitro percutaneous penetration studies were carried out according to the methods of Example I with formulations of a topical gel containing about 3% Azone and various concentrations of MTX. The results, shown in Table 2, shows that there is a dose-dependent increase in MTX penetration in vitro for this vehicle. It also shows that Azone is an effective penetration agent.

Table 2

| % Methotrexate | Cumulative Penetration 48 hr (µg) | Epidermal Content (µg) |
|---|---|---|
| 0.1% | 0.6±0.2 | 3.9± 0.3 |
| 0.5% | 7.5±1.4 | 24.1± 8.1 |
| 1.0% | 18.7±6.1 | 36.0±11.7 |

Example IV

In Vivo Animal Study of MTX and Azone Formulation

The formulations of Example III were tested for effect on in vivo DNA synthesis in the mini-pig model. Mini-pig skin closely resembles human skin and the effect of a drug on epidermal cell proliferation is a recognized test for the effectiveness of therapeutic protocols for hyperproliferative skin disease.

Mini-pigs were obtained from Charles River Laboratories, Inc. (Wilmington, MA). Drug preparations were applied to small areas of dorsal skin (ca 1 inch square) according to the selected application schedules. Six hours following the last drug application, tritiated thymidine was injected into the drug treatment sites for autoradiographic assessment of epidermal proliferation as measured by epidermal labeling index. Weinstein & and Van Scott, (1965) J. Invest. Derm. 44:413

A vehicle containing various concentrations of MTX was applied daily for 7 days. Tritiated deoxyuridine was injected into the treatment sites on day 7, as well as in untreated control area. DNA synthesis was measured as described in Example II.

The results of the test are shown in Table 3. Topical application of MTX, at both 0.5% and 1.0% suppressed epidermal DNA synthesis, compared to the vehicle alone. A slight decrease in labeling intensity was also seen, reflecting a decrease in the rate of DNA synthesis compared to the vehicle control.

Table 3

| % Methotrexate | Epidermal Labeling Index (%) |
|---|---|
| 0 (no Rx) | 8.1 ± 1.0 |
| 0 (vehicle) | 11.2 ± 1.6 |
| 0.5% | 0.0 ± 0.0 |
| 1.0% | 0.8 + 1.4 |

Example V

In Vitro Comparison of MTX in Various Formulations

Employing the methods described in Example I, various vehicles containing 2% by weight of tritiated MTX were tested in vitro on human skin. The results are shown in Table 4 below. As can be seen, water was not an effective vehicle. At 24 hr, percutaneous penetration was best with Vehicle N, vehicles containing N-methylpyrrolidone (NMP), and Azone (1-dodecyl-azacycloheptan-2-one). The epidermal content at 48 hr was also highest for these vehicles.

Table 4

| Vehicle | Percutaneous Penetration (µg) 1 hr | 24 hr | 48 hr | Stratum Corneum Content (48 hr) µg (±S.D.) | Epidermal Content (48 hr) µg (±S.D.) |
|---|---|---|---|---|---|
| $H_2O$ (pH 8.5) | 1±1 | 6±2 | 15±5 | 9±5 | 0 |
| $C_{10}MSO$ (25% $H_2O$) | 1±1 | 12±10 | 36±28 | 21±5 | 1±0 |
| Vehicle N | 1±0 | 50±11 | 124±10 | 16±8 | 2±1 |
| NMP (38%) (EtoH: 43%: $H_2O$ 19%) | 1±1 | 27±5 | 46±21 | 34±14 | 2±1 |
| Azone | 1±0 | 69±23 | 158±60 | 85±19 | 3±1 |

Example VI

Various MTX- and Azone- Containing Formulations In Vivo

Additional studies with the mini-pig model, as described in Example IV, were conducted to test various carriers with different concentrations of MTX and Azone. Formulations were applied daily for 7 days. Three sites per treatment area were injected with $^3$H-UdR. Autoradiographs were scored at 4 wk. Techniques as described in Examples I and II were employed to measure percutaneous penetration and DNA synthesis.

The results are shown in Table 5. As can be seen, numerous formulations containing concentrations of MTX ranging from 0.5% to 1% and concentrations of Azone ranging from 3% to 10% effectively penetrated the epidermis and significantly inhibited DNA synthesis. The increase in DNA synthesis seen in formulations containing 1% MTX and 10% Azone is probably due to irritation to the epidermis caused by the vehicle.

Table 5

| Carrier | % MTX | % Azone | Labeling Index (X ± S.D.) | 48 hr PC (µg) |
|---|---|---|---|---|
| Isopropanol: -- | | 0% | 10% | 18.8 ± 8.9 |
| $H_2O$ | 1% | 10% | 6.8 ± 11.8 | 74.3 |
| Isopropanol: $H_2O$:HCl | 1% | 10% | 5.8 ± 4.3 | 106 |
| Gel | 0% | 7% | 13.9 ± 8.2 | -- |
| Gel | 1% | 7% | 1.8 ± 3.1 | 24.5 |
| HCl Gel | 1% | 7% | 0.0 ± 0.0 | 25.3 |
| HCl Gel | 0.5% | 7% | 0.8 ± 1.4 | 8.1 |
| Gel | 0% | 3% | 11.2 ± 1.6 | 0 |
| Gel | 1% | 3% | 0.5 ± 0.9 | 26.1 |
| HCl Gel | 1% | 3% | 0.8 ± 1.4 | 25.6 |
| HCl Gel | 0.5% | 3% | 0.0 ± 0.0 | 11.5 |
| Control (no treatment) | -- | -- | 8.1 ± 1.0 | |

Example VII

In Vitro Percutaneous Penetration Studies of MTX in Various Vehicles

Various vehicle formulations were evaluated in vitro at various MTX concentrations for penetration of MTX

into the epidermis. Penetration was evaluated as described in Example I. Unless otherwise noted, all vehicles were a gel formulation that contained 3% Azone. The results of these studies are shown in Table 6. The data demonstrates that there is a dose-dependent increase in MTX penetration in all of the vehicles tested. The maximum penetration was obtained with the standard vehicle comprised of 10% Azone, 45% isopropanol, and 45% water. Significant penetration was obtained with all the vehicles, with those containing 1% MTX all showing comparable penetration.

Table 6

| Formulations | % MTX | Cumulative Penetration 48 hr (µg) | Epidermal/Stratum Corneum Content (µg) |
|---|---|---|---|
| Azone Gel #1 | 0.1 | 1.4 ± 0.3 | 14.5 ± 3.0 |
| Azone Gel #1 | 0.5 | 11.7 ± 3.1 | 58.9 ± 20.0 |
| Azone Gel #1 | 1.0 | 24.7 ± 3.5 | 49.4 ± 21.7 |
| Azone Gel #2 | 0.1 | 1.4 ± 0.3 | 1.3 ± 0.6 |
| Azone Gel #2 | 0.5 | 7.9 ± 1.5 | 5.1 ± 2.8 |
| Azone Gel #2 | 1.0 | 19.4 ± 7.9 | 12.0 ± 4.6 |
| Standard Azone:IPA:$H_2O$ 10:45:45 | 1.0 | 84.8 ± 9.6 | 71.8 ± 52.0 |

Since modifications will be apparent to those skilled in the art, it is intended that this invention be limited only by the scope of the appended claims.

## Claims

**Claims for the following Contracting States: AT, BE, CH, DE, FR, GB, IT, LI, NL, SE**

1. A composition for topical application that is therapeutic for a hyperproliferative epithelial disease comprising:

(a) a topical carrier containing an effective amount of a skin penetration agent that allows methotrexate to penetrate to the basal epidermal cells of a mammal in sufficient quantity to inhibit DNA synthesis in said basal epidermal cells, wherein said skin penetration agent is a compound having the formula:

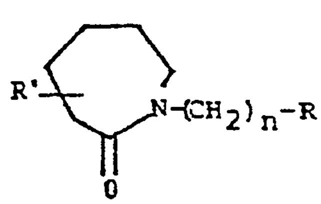

wherein R′ is selected from H and alkyls having 1-4 carbon atoms, R is selected from an alkyl of 1-18 carbon atoms and phenyl, and n is 0 or a positive integer from 1-10; and

(b) a therapeutically effective amount of a drug selected from methotrexate; 3′,5′-dichloromethotrexate; a mono- or dialkyl ester of methotrexate; and a mono- or dialkyl ester of 3′,5′-dichloromethotrexate.

2. A composition according to claim 1 wherein said drug is present in an amount from 0.1% to 10% by weight of the composition.

3. A composition according to claim 1 or 2 wherein said drug is methotrexate.

4. A composition according to claim 3 wherein methotrexate is at a concentration of from 0.1 to 10% by weight of the composition.

5. A composition according to claim 3 wherein methotrexate is at a concentration of from 0.5% to 5% by weight of the composition.

6. A composition according to any one of the preceding claims wherein said skin penetration agent is 1-dodecyl-azacycloheptan-2-one.

7. A composition according to claim 6 wherein the concentration of 1-dodecyl-azacycloheptan-2-one is in the range of 0.1% to 10% by weight of the topical carrier.

8. A composition according to claim 6 wherein the concentration of 1-dodecyl-azacycloheptan-2-one is in the range from 0.1% to 5% by weight of the topical carrier.

9. A composition according to any one of the preceding claims wherein said topical carrier is a viscous topical medicament carrier.

10. A composition according to any one of the preceding claims for use in a method of treatment or therapy of a hyperproliferative epithelial disease in a mammal.

**Claims for the following Contracting State: ES**

1. A method for preparing a composition for topical application that is therapeutic for a hyperproliferative epithelial disease comprising formulating:

(a) a topical carrier containing an effective amount of a skin penetration agent that allows methotrexate to penetrate to the basal epidermal cells of a mammal in sufficient quantity to inhibit DNA synthesis in said basal epidermal cells, wherein said skin penetration agent is a compound having the formula:

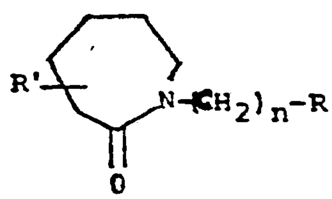

wherein R′ is selected from H and alkyls having 1-4 carbon atoms, R is selected from an alkyl of 1-18 carbon atoms and phenyl, and n is 0 or a positive integer from 1-10; and

(b) a therapeutically effective amount of a drug selected from methotrexate; 3′,5′-dichloromethotrexate; a mono- or dialkyl ester of methotrexate; and a mono- or dialkyl ester of 3′,5′-dichloromethotrexate.

2. A method according to claim 1 wherein said drug is present in an amount from 0.1% to 10% by weight of the composition.

3. A method according to claim 1 or 2 wherein said drug is methotrexate.

4. A method according to claim 3 wherein methotrexate is at a concentration of from 0.1 to 10% by weight of the composition.

5. A method according to claim 3 wherein methotrexate is at a concentration of from 0.5% to 5% by weight of the composition.

6. A method according to any one of the preceding claims wherein said skin penetration agent is 1-dodecyl-azacycloheptan-2-one.

7. A method according to claim 6 wherein the concentration of 1-dodecyl-azacycloheptan-2-one is in the range of 0.1% to 10% by weight of the topical carrier.

8. A method according to claim 6 wherein the concentration of 1-dodecyl-azacycloheptan-2-one is in the range from 0.1% to 5% by weight of the topical carrier.

9. A method according to any one of the preceding claims wherein said topical carrier is a viscous topical medicament carrier.

10. A composition prepared by a method according to any one of the preceding claims for use in a method of treatment or therapy of a hyperproliferative epithelial disease in a mammal.

## Patentansprüche

### Patentansprüche für folgende Vertragsstaaten: AT, BE, CH, DE, FR, GB, IT, LI, NL, SE

1. Mittel zur topischen Anwendung, das therapeutisch wirksam ist gegen hyperproliferative Epithelerkrankungen, umfassend:

(a) einen topischen Träger, enthaltend eine wirksame Menge eines Hautdurchdringungsmittels, das es erlaubt, daß Methotrexat zu den basalen Epidermiszellen eines Säugetiers in ausreichender Menge durchdringt, um die DNA-Synthese in den basalen Epidermiszellen zu hemmen, wobei das Hautdurchdringungsmittel eine Verbindung ist der Formel

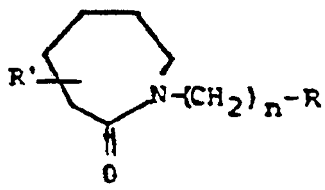

in der R′ ausgewählt ist aus H und Alkylgruppen mit 1-4 Kohlenstoffatomen, R ausgewählt ist aus einer Alkylgruppe mit 1-18 Kohlenstoffatomen und Phenyl, und n 0 oder eine positive ganze Zahl von 1-10 ist, und

(b) eine therapeutisch wirksame Menge eines Arzneimittels, ausgewählt aus Methotrexat, 3′5′-Dichlormethotrexat, einem Mono- oder Dialkylester von Methotrexat und einem Mono- oder Dialkylester von 3′,5′-Dichlormethotrexat.

2. Mittel nach Anspruch 1, wobei das Arzneimittel in einer Menge von 0,1 bis 10 Gew.-% des Mittels vorhanden ist.

3. Mittel nach Anspruch 1 oder 2, wobei des Arzneimittel Methotrexat ist.

4. Mittel nach Anspruch 3, wobei das Methotrexat in einer Konzentration von 0,1 bis 10 Gew.-% des Mittels vorliegt.

5. Mittel nach Anspruch 3, wobei Methotrexat in einer Konzentration von 0,5 bis 5 Gew.-% des Mittels vorliegt.

6. Mittel nach einem der vorangehenden Ansprüche, wobei das Hautdurchdringungsmittel 1-Dodecyl-azacycloheptan-2-on ist.

7. Mittel nach Anspruch 6, wobei die Konzentration an 1-Dodecyl-azacycloheptan-2-on im Bereich von 0,1 bis 10 Gew.-% des topischen Trägers liegt.

8. Mittel nach Anspruch 6, wobei die Konzentration an 1-Dodecyl-azacycloheptan-2-on im Bereich von 0,1 bis 5 Gew.-% des topischen Trägers liegt.

9. Mittel nach einem der vorangehenden Ansprüche, wobei der topische Träger ein viskoser topischer Arzneimittelträger ist.

10. Mittel nach einem der vorangehenden Ansprüche zur Verwendung bei einem Verfahren zur Behandlung oder Therapie einer hyperproliferativen Epithelerkrankung bei Säugetieren.

### Patentansprüche für folgenden Vertragsstaat: ES

1. Verfahren zur Herstellung eines Mittels zur topischen Anwendung, das therapeutisch wirksam ist gegen hyperproliferative Epithelerkrankungen, unfassend das Zusammenbringen von:

(a) einen topischen Träger, enthaltend eine wirksame Menge eines Hautdurchdringungsmittels, das es erlaubt, daß Methotrexat zu den basalen Epidermiszellen eines Säugetiers in ausreichender Menge durchdringt, um die DNA-Synthese in den basalen Epidermiszellen zu hemmen, wobei das Hautdurchdringungsmittel eine Verbindung ist der Formel

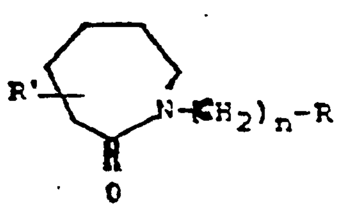

in der R' ausgewählt ist aus H und Alkylgruppen mit 1-4 Kohlenstoffatomen, R ausgewählt ist aus einer Alkylgruppe mit 1-18 Kohlenstoffatomen und Phenyl, und n 0 oder eine positive ganze Zahl von 1-10 ist, und

(b) eine therapeutisch wirksame Menge eines Arzneimittels, ausgewählt aus Methotrexat, 3'5'-Dichlormethotrexat, einem Mono- oder Dialkylester von Methotrexat und einem Mono- oder Dialkylester von 3',5'-Dichlormethotrexat.

2. Verfahren nach Anspruch 1, wobei das Arzneimittel in einer Menge von 0,1 bis 10 Gew.-% des Mittels vorhanden ist.

3. Verfahren nach Anspruch 1 oder 2, wobei das Arzneimittel Methotrexat ist.

4. Verfahren nach Anspruch 3, wobei das Methotrexat in einer Konzentration von 0,1 bis 10 Gew.-% des Mittels vorliegt.

5. Verfahren nach Anspruch 3, wobei Methotrexat in einer Konzentration von 0,5 bis 5 Gew.-% des Mittels vorliegt.

6. Verfahren nach einem der vorangehenden Ansprüche, wobei das Hautdurchdringungsmittel 1-Dodecyl-azacycloheptan-2-on ist.

7. Verfahren nach Anspruch 6, wobei die Konzentration an 1-Dodecyl-azacycloheptan-2-on im Bereich von 0,1 bis 10 Gew.-% des topischen Trägers liegt.

8. Verfahren nach Anspruch 6, wobei die Konzentration an 1-Dodecyl-azacycloheptan-2-on im Bereich von 0,1 bis 5 Gew.-% des topischen Trägers liegt.

9. Verfahren nach einem der vorangehenden Ansprüche, wobei der topische Träger ein viskoser topischer Arzneimittelträger ist.

10. Mittel, hergestellt nach einem Verfahren nach einem der vorangehenden Ansprüche zur Verwendung bei einem Verfahren zur Behandlung oder Therapie einer hyperproliferativen Epithelerkrankung bei Säugetieren.

## Revendications

### Revendications pour les Etats contractants suivants: AT, BE, CH, DE, FR, GB, IT, LI, NL, SE

1. Composition pour application locale, qui est thérapeutique pour une maladie épithéliale hyperproliférative, comprenant :

(a) un véhicule topique contenant une quantité efficace d'un agent de pénétration de la peau qui permet au méthotrexate de pénétrer dans les cellules épidermiques basales d'un mammifère, en quantité suffisante pour inhiber la synthèse d'ADN dans lesdites cellules épidermiques basales, dans laquelle ledit agent de pénétration de la peau est un composé de formule :

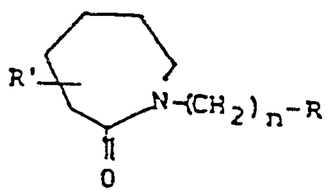

dans laquelle R' est choisi parmi H et les groupes alkyle comportant 1-4 atomes de carbone, R est choisi parmi un groupe alkyle de 1-18 atomes de carbone et un groupe phényle, et n est nul ou est un nombre entier positif de 1-10; et

(b) une quantité thérapeutiquement efficace d'un médicament choisi parmi le méthotrexate, le 3',5'dichlorométhotrexate, un ester mono- ou dialkylique de méthotrexate, et un ester mono- ou dialkylique de 3',5'dichlorométhotrexate.

2. Composition selon la revendication 1, dans laquelle ledit médicament est présent en une quantité de 0,1% à 10% du poids de la composition.

3. Composition selon la revendication 1 ou 2, dans laquelle ledit médicament est le méthotrexate.

4. Composition selon la revendication 3, dans laquelle le méthotrexate est présent à une concentration de 0,1 à 10% du poids de la composition.

5. Composition selon la revendication 3, dans laquelle le méthotrexate est présent à une concentration de 0,5 à 5% du poids de la composition.

6. Composition selon l'une quelconque des revendications précédentes, dans laquelle ledit agent de pénétration de la peau est la 1-dodécyl-azacycloheptane-2-one.

7. Composition selon la revendication 6, dans laquelle la concentration en 1-dodécyl-azacycloheptane-2-one est dans l'intervalle de 0,1% à 10% du poids du véhicule topique.

8. Composition selon la revendication 6, dans laquelle la concentration en 1-dodécyl-azacycloheptane-2-one est dans l'intervalle de 0,1% à 5% du poids du véhicule topique.

9. Composition selon l'une quelconque des revendications précédentes, dans laquelle ledit véhicule topique est un véhicule topique visqueux pour médicament.

10. Composition selon l'une quelconque des revendications précédentes, à utiliser dans un procédé de traitement ou dans une thérapeutique d'une maladie épithéliale hyperproliférative chez un mammifère.

**Revendications pour l'Etat contractant suivant: ES**

1. Procédé de préparation d'une composition pour application locale, qui est thérapeutique pour une maladie épithéliale hyperproliférative, comprenant la formulation :

(a) d'un véhicule topique contenant une quantité efficace d'un agent de pénétration de la peau qui permet au méthotrexate de pénétrer dans les cellules épidermiques basales d'un mammifère, en quantité suffisante pour inhiber la synthèse d'ADN dans lesdites cellules épidermiques basales, dans lequel ledit agent de pénétration de la peau est un composé de formule :

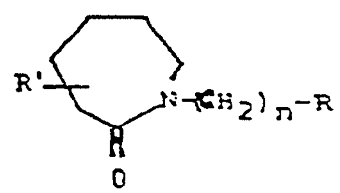

dans laquelle R' est choisi parmi H et les groupes alkyle comportant 1-4 atomes de carbone, R est choisi parmi un groupe alkyle de 1-18 atomes de carbone et un groupe phényle, et n est nul ou est un nombre entier positif de 1-10; et

(b) d'une quantité thérapeutiquement efficace d'un médicament choisi parmi le méthotrexate, le 3',5'-dichlorométhotrexate, un ester mono- ou dialkylique de méthotrexate, et un ester mono- ou dialkylique de 3',5'-dichlorométhotrexate.

2. Procédé selon la revendication 1, dans lequel ledit médicament est présent en une quantité de 0,1 à 10% du poids de la composition.

3. Procédé selon la revendication 1 ou 2, dans lequel ledit médicament est le méthotrexate.

4. Procédé selon la revendication 3, dans lequel le méthotrexate est présent à une concentration de 0,1 à 10% du poids de la composition.

5. Procédé selon la revendication 3, dans lequel le méthotrexate est présent à une concentration de 0,5 à 5% du poids de la,composition.

6. Procédé selon l'une quelconque des revendications précédentes, dans lequel ledit agent de pénétration de la peau est la 1-dodécyl-azacycloheptane-2-one.

7. Procédé selon la revendication 6, dans lequel la concentration en 1-dodécyl-azacycloheptane-2-one est dans l'intervalle de 0,1% à 10% du poids du véhicule topique.

8. Procédé selon la revendication 6, dans lequel la concentration en 1-dodécyl-azacycloheptane-2-one est dans l'intervalle de 0,1% à 5% du poids du véhicule topique.

9. Procédé selon l'une quelconque des revendications précédentes, dans lequel ledit véhicule topique est un véhicule topique visqueux pour médicament.

10. Composition préparée par un procédé selon l'une quelconque des revendications précédentes, à utiliser dans un procédé de traitement ou dans une thérapeutique d'une maladie épithéliale hyperproliférative chez un mammifère.